# EUROPEAN PATENT APPLICATION

(11) **EP 4 464 767 A1**
(43) Date of publication of application: **20.11.2024**
(21) Application number: 23740526.1
(22) Date of filing: 16.01.2023
(51) Int. Cl.: C12M 1/00, C12M 3/00, G01N 33/50, C12N 5/077, C12N 5/071, C12M 1/34

(54) **HUMAN ORGAN-ON-A-CHIP HAVING REVERSE MICRO-WEIR STRUCTURE AND USES THEREOF**

(30) Priority: 14.01.2022 KR 20220006035; 26.01.2022 KR 20220011109
(71) Applicant: Humanase, Pocheon-si Gyeonggi-do 11160 (KR)
(72) Inventor: BAEK, Sangwon, Seongnam-si, Gyeonggi-do 13372 (KR); PARK, Sungbin, Seongnam-si, Gyeonggi-do 13357 (KR)
(74) Representative: Kador & Partner Part mbB
(86) International application number: PCT/KR2023/000715
(87) International publication number: WO 2023/136683

(57) **Abstract**

The present invention relates to an organ-on-a-chip having a reverse micro-weir structure and uses thereof. Using the organ-on-a-chip for evaluating drug efficacy and toxicity provided by the present invention makes it possible to overcome the limitations of conventional two-dimensional in-vitro cell culture methods that cannot mimic the human microenvironment and the inaccuracies due to differences between species in animal testing, and to derive more accurate efficacy and toxicity results for cells treated with drugs. Accordingly, as the organ-on-a-chip of the present invention is used as an alternative test method to animal testing, the organ-on-a-chip can drastically reduce the cost and time required for new drug development and drug screening and can also be effectively used to conduct research on the cellular microenvironment, other organ-on-a-chips, and drug metabolism mechanisms and to develop new drugs.

## Description

### [Technical Field]

The present invention relates to a human organ-on-chip having an inverted micro-weir structure and use thereof.

### [Background Art]

Drug development is time-consuming, intensive, and costly, but only a few drugs that have been developed are approved. The costs and time for achieving regulatory approval for a single drug are greater than $2.5 billion and between 10 and 15 years, respectively. To address these issues, numerous animal- or cell-based experiments have been typically conducted during the initial stages of drug development.

Animal-based experiments are expensive, require a long time, and are not free from ethical issues, and in many cases, animal experimental results cannot be applied to humans due to the limitations of inter-species differences, such as the existence of different ion channels from humans and pharmacokinetic differences. Moreover, even drugs that have passed clinical trials through animal experiments are often withdrawn due to side effects. In cell-based experiments, cells are usually cultured under two-dimensional conditions. While two-dimensional cell culture models are advantageous in terms of ease of handling and high throughput, they have limitations in drug efficacy and toxicity evaluation since cells lose their inherent functionality during two-dimensional culture, resulting in incorrect evaluation results. In human organs, cells exist in a three-dimensional structure environment with extracellular matrix (ECM), and continuously receive nutrients and oxygen through diffusion from capillaries and diffuse and discharge the resultant wastes, metabolites, and carbon dioxide through blood vessels. For these reasons, there is a need for an efficient drug efficacy and toxicity evaluation platform that reflects the structural, physiological, and environmental characteristics of actual organs.

Organ-on-a-chips (OoCs) or human organ-on-a-chips are systems that mimic the internal microenvironment structures of specific organs in the human body and allow cells constituting the corresponding organs to be cultured in micro-chambers, thereby implementing the characteristics thereof. Therefore, organ-on-a-chips are an effective alternative that can overcome the limitations of animal testing and are expected to be a pre-clinical new drug development platform by implementing physiological activity at the tissue or organ level.

One of these human organ-on-a-chips is an organ-on-a-chip using cancer cells, which is used to achieve purposes, such as testing the pharmacological activity of anticancer drug candidates and identifying mechanisms of cancer metastasis. For example, Korean Patent No. 10-1437149 discloses a cancer cell culture chip in the form where culture media concentrated by fibroblasts are supplied to cancer cells. Korean Patent No. 10-1621211 discloses a microfluidic chip for anticancer drug testing, capable of checking whether cancer cells infiltrate vascular cells. Korean Patent No. 10-1709312 discloses a hydrogel-based microfluidic chip for cell co-culture, capable of co-culturing vascular endothelial cells and cancer cells. Korean Patent No. 10-1701607 discloses a microfluidic chip for screening anticancer drug-resistant cells by inducing a continuous concentration gradient among cell culture chambers. Korean Patent No. 10-1784703 discloses a three-dimensional culture chip comprising a hydrogel using bladder submucosa matrix to create a biomimetic cell culture environment. Korean Patent No. 10-1913777 discloses a cell chip for evaluating anticancer drug efficacy based on a three-dimensional mesh-type microstructure, capable of reproducing the original characteristics of cancer cells and implementing similar activities to the in vivo environment by culturing three-dimensional cubic cancer cells using a mesh-type microstructure having a three-dimensional pyramid structure. Korean Patent Publication No. 10-2021-0014464 discloses a blood vessel-mimicking microfluidic chip for cell co-culture, capable of mimicking normal vascular tissue, cancer tissue, and cancer-metastatic vascular tissue. Also, a 3-channel microfluidic system mimicking a lymph vessel-tissue-blood vessel (LTB) structure is disclosed, the system being capable of identifying intercellular interactions in the cancer microenvironment under various extracellular stimuli, such as inflammatory cytokines, stromal reaction, hypoxia, and nutrient deficiency during lymphatic metastasis (Cho H-Y, Choi J-H, Kim K-J, Shin M, and Choi J-W (2021) Microfluidic System to Analyze the Effects of Interleukin 6 on Lymphatic Breast Cancer Metastasis; Front. Bioeng. Biotechnol. 8:611802).

### [Disclosure]

### [Technical Problem]

The present inventors developed a novel organ-on-a-chip capable of culturing cancer cells, derived from humans, in an environment similar to the in vivo environment.

### [Technical Solution]

An aspect of the present invention is to provide an organ-on-a-chip including a media supply section for supplying media to cells, a cell supply section containing fixed cells, and an inverted micro-weir structure provided therebetween.

Another aspect of the present invention is to provide a method for evaluating the efficacy of an anticancer drug on cancer cells by using the organ-on-a-chip.

### [Advantageous Effects]

The use of the organ-on-a-chip provided by the present invention can derive more accurate efficacy of an anticancer drug on cancer cells or the like. Therefore, the present invention can be used as an alternative method to animal testing to thereby significantly reduce the cost and time required for new drug development and drug screening and can be helpfully used in research on the intracellular microenvironment and research on other organ-on-a-chips.

### [Brief Description of Drawings]

FIG. 1 shows a three-dimensional view and a microstructure view of an organ-on-a-chip provided by the present invention and an overall schematic view featuring all elements in the chip.
FIG. 2 is a three-dimensional view of the organ-on-a-chip provided by the present invention.
FIG. 3 is a plane view of the organ-on-a-chip provided by the present invention.
FIG. 4 is a partial enlarged view of a cell chamber and media channels included in the organ-on-a-chip provided by the present invention.
FIG. 5 presents partial enlarged views of an inverted micro-weir structure included in the organ-on-a-chip provided by the present invention.
FIG. 6 presents images comparing the degree of damage to breast cancer cells (MCF-7) after the breast cancer cells were injected into the organ-on-a-chip provided by the present invention, cultured for 2 days, and then treated with the anticancer drug doxorubicin, when compared with the control group.
FIG. 7 presents images comparing the degree of damage to cervical cancer cells (HeLa) after the cervical cancer cells were injected into the organ-on-a-chip provided by the present invention, cultured for 2 days, and then treated with the anticancer drug doxorubicin, when compared with the control group.
FIG. 8 presents images comparing the degree of damage to liver cancer cells (HepG2) after the liver cancer cells were injected into the organ-on-a-chip provided by the present invention, cultured for 2 days, and then treated with the anticancer drug doxorubicin, when compared with the control group.

### [Detailed Description of the Invention]

In an aspect of the present invention, there is provided an organ-on-a-chip, including: (a) a media supply section comprising: a media supply chamber; a media recovery chamber; and a media channel communicating with each of the chambers; (b) a cell supply section comprising: a cell inlet provided at one end; a residue outlet provided at the other end; and a cell chamber provided between the cell inlet and the residue outlet and extending adjacent to the media channel in the same direction; and (c) an inverted micro-weir structure provided in an area selected from the group consisting of an adjacent area between the media channel and the cell chamber, an adjacent area between the cell chamber and the residue outlet, and a combination thereof.

The term "inverted micro-weir structure" used herein refers to a partial blocking member provided in an adjacent area between the media channel and the cell chamber or an adjacent area between the cell chamber and the residue outlet. Specifically, the inverted micro-weir structure is meant to be a partial blocking member that blocks a partial space of an adjacent area between the media channel and the cell chamber or an adjacent area between the cell chamber and the residue outlet to allow the movement of a liquid component while preventing the movement of cells. The partial blocking member was named "inverted micro-weir structure" in the sense that it is a weir-shaped structure connected upside down to the ceiling. To take a specific example, as for the adjacent area between the media channel and the cell chamber, an inverted micro-weir structure may be provided that extends along the adjacent area in the same direction, wherein the upper end of the inverted micro-weir structure is combined with the upper end of the adjacent area, and the lower end of the inverted micro-weir structure is spaced from the bottom of the adjacent area. To take another example, as for the adjacent area between the cell chamber and the residue outlet, an inverted micro-weir structure may be provided that extends along the adjacent area in the same direction, wherein the upper end of the inverted micro-weir structure is combined with the upper end of the adjacent area and the lower end of the inverted micro-weir structure is spaced from the bottom of the adjacent area.

As defined above, the inverted micro-weir structure included in the organ-on-a-chip provided by the present invention may be configured such that the upper end of the inverted micro-weir structure is combined with the upper end of the adjacent area and the lower end of the inverted micro-weir structure is spaced from the bottom of the adjacent area, thereby allowing the passage of a liquid component through the spaced site while preventing the passage of cells or cured cells therethrough.

The shape of the inverted micro-weir structure is not particularly limited as long as it exhibits the foregoing functionality. In an embodiment, the cross-section of the inverted micro-weir structure may be rectangular or arc-shaped, and because this structure allows for free flow of media in and out of the cell chamber while preventing the cells from leaving the cell chamber. Additionally, the gap of the spaced site included in the lower end is not particularly limited as long as it can prevent the passage of cells. In one embodiment, the gap may be 5 to 20 µm; in another embodiment, the gap may be 7 to 15 µm; and in still another embodiment, the gap may be 10 µm.

If the gap of the spaced site is less than 5 µm, a liquid component cannot pass between the lower end of the inverted micro-weir structure and the bottom of the adjacent area due to surface tension. If the gap of the spaced site is more than 20 µm, cells may move through the spaced site to leave the cell chamber.

Alternatively, the inverted micro-weir structure, when provided in the adjacent area between the media channel and the cell chamber, may be configured such that the structure extends along the adjacent area in the same direction and allows the inter-flow of media between the media channel and the cell chamber through the spaced site of the lower end while preventing the passage of the cells therethrough.

Additionally, the inverted micro-weir structure, when provided in the adjacent area between the cell chamber and the residue outlet, may be configured such that the structure is in the form of an arc curved toward the residue outlet and allows the discharge of residue from the cell chamber to the residue outlet through the spaced site of the lower end while preventing the passage of the cells therethrough.

In the organ-on-a-chip provided by the present invention, the inverted micro-weir structure, which is an improved partial blocking member described above, shows the same effects as conventional micro-pillar structures in that the inverted micro-weir structure serves to prevent the movement of cells by partially opening the adjacent area between the media channel and the cell chamber, rather than completely opening the adjacent area.

Nevertheless, the inverted micro-weir structure has a simple shape compared with the micro-pillar structures having an elaborate and complicated shape, and thus has the advantages of reducing the production cost of a mold used in its manufacture and improving the yield of products obtained from the mold.

Meanwhile, as for micro-pillar structures provided in conventional endomyocardial-level biomimetic heart-on-a-chips, the formation of boundaries needs to be induced by surface tension at the sites where the round corners of the micro-pillar structures begin, so that when a cell mixture is injected into the cell chamber, the cell mixture enters only the cell chamber but not the culture channel. To this end, a post-treatment process needs to be essentially conducted in an oven at 80°C for 48 hours, resulting in increased production costs and time. However, the inverted micro-weir structure provided by the present invention is structurally designed so that cells cannot pass through the inverted micro-weir structure, and this structure eliminates the need to form boundaries by surface tension and there is no need to perform a post-processing process therefor.

Therefore, the organ-on-a-chip including the inverted micro-weir structure provided by the present invention can simplify the manufacturing process, leading to an improvement in productivity.

A material for the entire structure of the organ-on-a-chip provided by the present invention is not particularly limited as long as it does not hinder or adversely affect the fixation and culture of cells. Examples of the material may include polycaprolactone (PCL), poly(dimethylsiloxane) (PDMS), polylactic acid (PLA), polyglycolic acid (PGA), polydioxanone (PDO), and the like. In another embodiment, the material may be PDMS or the like, which is optically transparent, highly durable, bio-friendly, and flexible.

The media supply chamber included in the organ-on-a-chip provided by the present invention serves to store media for cell culture, which is to be supplied to the cell chamber through the inverted micro-weir structure, and to supply the stored media to the cells through the media channel. Additionally, in order to more precisely evaluate the efficacy of an anticancer drug against cancer cells, the media supply chamber may be used as a culture container for culturing liver cells therein.

The media recovery chamber included in the organ-on-a-chip provided by the present invention is a place where the media supplied from the media supply chamber is finally stored after passing through the media channel. The media recovery chamber serves to recover the media contaminated after cell culture and is used to prevent the contamination of surrounding environments due to the media contaminated with various metabolites and wastes secreted from the cells after cell culture.

The media channel included in the organ-on-a-chip provided by the present invention serves as a main media flow path through which the media flows from the media supply chamber to the media recovery chamber and, especially, the media channel serves to supply the media to cells located in the cell chamber and recover the media used for cell culture, through the inverted micro-weir structure. In the present invention, the media channel may be interpreted as a constituent element mimicking capillaries for supplying nutrients to cells existing in a living body. Therefore, the width of the media channel is not particularly limited as long as the media channel can mimic the functionality of capillaries. In an embodiment, the media channel may have a width of 20 to 40 µm; in another embodiment, the media channel may have a width of 25 to 35 µm; and in still another embodiment, the media channel may have a width of 30 µm.

Furthermore, vascular endothelial cells may be cultured inside the media channel to fabricate an organ-on-a-chip in a form that is more similar to a living environment.

The cell inlet included in the organ-on-a-chip provided by the present invention serves to introduce a cell mixture, which is to be supplied to the cell chamber. For example, the organ-on-a-chip may be configured such that the nozzle of a syringe may be connected to the cell inlet loaded with the cell mixture. The cell mixture may contain cells and an auxiliary ingredient for assisting the flow and fixation of the cells. The organ-on-a-chip of the present invention contains cells that are arranged and exist in a fixed form inside the cell chamber included therein, wherein for the injection of the cells to the cell chamber and the fixation of the cells inside the cell chamber, an auxiliary ingredient for assisting with the same is needed. The auxiliary ingredient, which mimics the extracellular matrix (ECM) existing in the microenvironment around cells, is essential for three-dimensional cell culture and serves to maintain the structures and functions of cultured cells to be similar to those in vivo. The auxiliary ingredient is not particularly limited as long as it can assist with the introduction and fixation of cells. In an embodiment, a hydrogel may be used, and in another embodiment, collagen hydrogel, alginate hydrogel, and GelMa hydrogel, or the like may be used. The hydrogel is mixed with cells to assist with three-dimensional culture of the cells and, additionally, imparts fluidity to the cells to allow the cells to move from the cell inlet to the cell chamber. The hydrogel may be cured under various conditions. For example, collagen hydrogel may be cured by being left in an incubator for 1 hour or longer to raise the temperature; alginate hydrogel may be cured by injecting a CaCl₂ solution into the media channel in place of the media; and GelMa hydrogel may be cured by ultraviolet ray irradiation. After the cell mixture moves to the cell chamber, the hydrogel contained in the cell mixture is cured, so that the cells contained within the cured hydrogel can be naturally fixed. The inherent porous characteristics of the hydrogel enable the delivery of external media to the cells through the hydrogel, thereby assisting with the culture of fixed cells.

As described above, the cell chamber included in the organ-on-a-chip provided by the present invention can store and fix the cell mixture supplied through the cell inlet as well as serve as a culture container for culturing the fixed cells.

In the present invention, the cell chamber may be interpreted as a constituent element mimicking the microstructure of cells existing in a living body. Therefore, the width of the cell chamber is not particularly limited as long as it can perform the fixation and culture of cells. In an embodiment, the width of the cell chamber may be 20 to 2000 µm; in another embodiment, the width may be 100 to 1000 µm; and in still another embodiment, the width may be 600 µm.

The cell chamber may be in the form of a passage communicating between the cell inlet and the residue outlet. An inverted micro-weir structure is provided within a portion of the cell chamber, which communicates with the residue outlet, so that the cells contained in the cell mixture supplied through the cell inlet remain in the cell chamber without moving to the residue outlet.

The type of cells that are injectable into the cell chamber is not particularly limited. To take one example, the cells may be selected from the group consisting of various types of solid cancer cells, cardiomyocytes, smooth muscle cells, striated muscle cells, kidney cells, liver cells, epithelial cells, nerve cells, stem cells, vascular endothelial cells, bone cells, scalp cells, spleen cells, lung cells, oral cells, skin cells, hair follicle cells, brain cells, spinal cord cells, and combinations thereof, and to take another example, the cells may be solid cancer cells, such as breast cancer cells, cervical cancer cells, or liver cancer cells.

The residue outlet included in the organ-on-a-chip provided by the present invention may serve to discharge contaminants, such as metabolites or wastes generated from the cells that are fixed and cultured in the cell chamber, to the outside. Such discharge of contaminants may be performed through the cell inlet in addition to the residue outlet. That is, the cell inlet, which has finished its role of introducing the cells into the cell chamber, may serve to discharge the contaminants, which are generated during the culture of the cells fixed in the cell chamber, to the outside.

The shape of the residue outlet is not particularly limited as long as it can serve to discharge contaminants to the outside, such as various metabolites and wastes secreted from cells contained in the cell mixture, like in the foregoing media recovery chamber. However, the residue outlet may be configured in a form to which a tube or the nozzle of a syringe can be coupled, in order to prevent the contamination of the cell's surrounding environment by the contaminants.

The organ-on-a-chip provided by the present invention basically includes at least one media supply section and at least one cell supply section, but may include multiple media supply sections and cell supply sections depending on the purpose of using the organ-on-a-chip.

For example, the organ-on-a-chip may include multiple media supply sections and one cell supply section, one media supply section and multiple cell supply sections, or multiple media supply sections and multiple cell supply sections. In particular, if the organ-on-a-chip includes multiple media supply sections and one cell supply section, the media supply sections may be provided at both sides of the cell supply section. In such a case, inverted micro-weir structures may be formed between one cell chamber and multiple media channels, and in order to eliminate the interference between each media channel, each media channel may be configured to avoid adjacency, or even if adjacent, it may be configured to be completely isolated from each other.

According to an embodiment of the present invention, the organ-on-a-chip may include: two media supply chambers; two media recovery chambers; two media channels; one cell inlet; one residue outlet; one cell chamber adjacent to the media channels; and inverted micro-weir structures provided in adjacent areas between the cell chamber and the media channels and in an adjacent area between the cell chamber and the residue outlet.

Culturing desired cells on the organ-on-a-chip provided by the present invention enables the completion of cell culture within about 7 days and the characterization of the cultured cells. For example, in the culture of breast cancer cells, the administration of a drug into the media supply chamber upon the completion of cell culture may result in the death of the breast cancer cells due to the efficacy of the drug. To take another example, in the culture of cervical cancer cells, the administration of a drug into the media supply chamber upon the completion of cell culture may result in the death of the cervical cancer cells due to the efficacy of the drug. To take still another example, in the culture of liver cancer cells, the administration of a drug into the media supply chamber upon the completion of cell culture may result in the death of the liver cancer cells due to the efficacy of the drug.

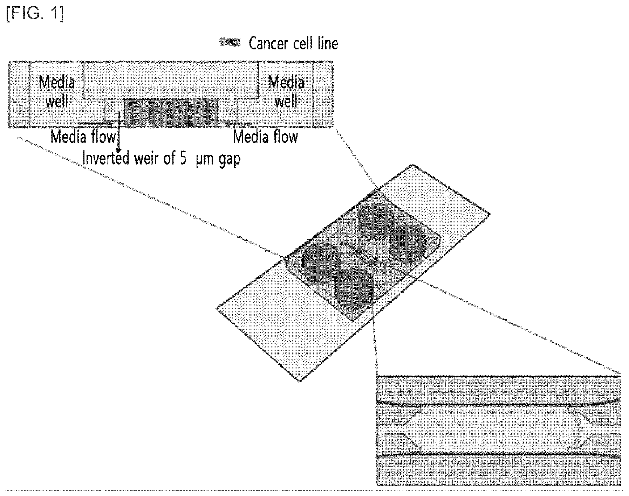

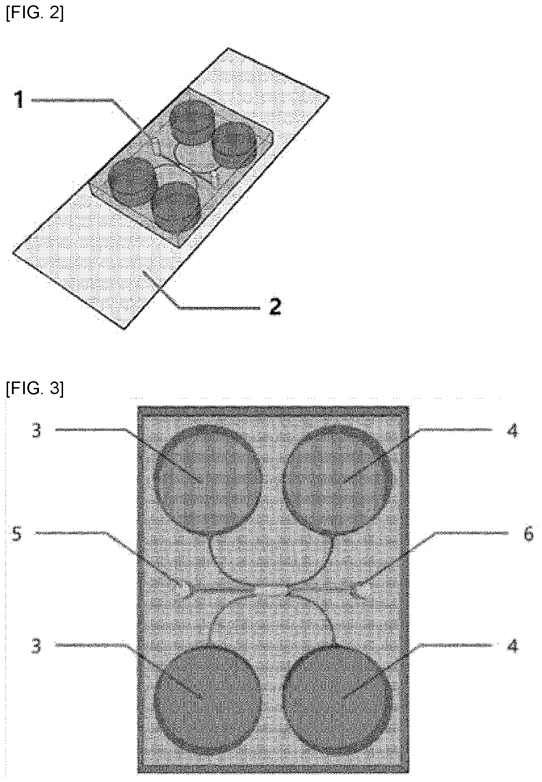

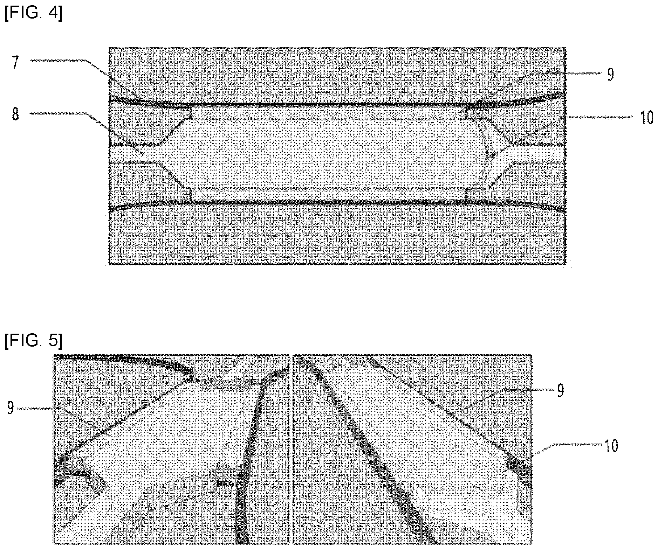

Hereinafter, the structure of an organ-on-a-chip provided by the present invention will be described in more detail with reference to FIGS. 1 to 5.

An organ-on-a-chip 2 provided by the present invention is in the form of a structure that is provided on a slide glass 1. Two sets of media supply sections each composed of a media supply chamber 3, a media recovery chamber 4, and a media channel 7 are provided at both sides of a cardiomyocyte supply section composed of a cell inlet 5, a residue outlet 6, and a cell chamber 8. The cell chamber 8 and the media channel 7 are distinguished from each other by an inverted micro-weir structure 9 provided in the adjacent area therebetween, and an inverted micro-weir structure 10 may also be provided in the adjacent area between the cell chamber 8 and the residue outlet 6.

In accordance with another aspect of the present invention, there is provided a method for evaluating the efficacy of a drug on cells.

One method for evaluating the efficacy of a drug on cells provided by the present invention includes: (a) culturing cells contained inside a cell chamber included in the organ-on-a-chip; (b) administering a desired drug to a media supply chamber included in the organ-on-a-chip; and (c) measuring a change of the cultured cells. The change of the cells is not particularly limited, but as an example, the change may be selected from the group consisting of whether cultured cells are damaged, whether cultured cells are grown, whether a secreted component is changed, whether the expression level of a gene is changed, whether the level of a protein is changed, and a combination thereof. As another example, the change of the cells may be whether cultured cancer cells are alive or dead.

Another method for evaluating the efficacy of a drug on cells provided by the present invention includes: (a) culturing liver cells contained inside a media supply chamber included in the organ-on-a-chip; (b) culturing cells contained inside the cell chamber included in the organ-on-a-chip; (c) administering a desired drug to the media supply chamber included in the organ-on-a-chip; and (d) measuring a change of the cultured cells.

According to one exemplary embodiment of the present invention, when breast cancer cells (MCF-7) were used as cells to be cultured, the attachment of the breast cancer cells was confirmed about 1 day after culture, and when the attached breast cancer cells were stabilized, the administration of a desired drug to the media supply chamber caused the death of the breast cancer cells by the efficacy of the drug.

As a result, the efficacy of the desired drug on the breast cancer cells could be evaluated by identifying the degree of death of the breast cancer cells.

According to another exemplary embodiment of the present invention, when cervical cancer cells (HeLa) were used as cells to be cultured, the attachment of the cervical cancer cells was confirmed about 1 day after culture, and when the attached cervical cancer cells were stabilized, the administration of a desired drug to the media supply chamber caused the death of the cervical cancer cells by the efficacy of the drug.

As a result, the efficacy of the desired drug on the cervical cancer cells could be evaluated by identifying the degree of death of the cervical cancer cells.

According to still another exemplary embodiment of the present invention, when liver cancer cells (HepG2) were used as cells to be cultured, the attachment of the liver cancer cells was confirmed about 1 day after culture, and when the attached liver cancer cells were stabilized, the administration of a desired drug to the media supply chamber caused the death of the liver cancer cells by the efficacy of the drug.

As a result, the efficacy of the desired drug on the liver cancer cells could be evaluated by identifying the degree of death of the liver cancer cells.

### [Mode for Carrying Out the Invention]

Hereinafter, the present invention will be described in detail with reference to exemplary embodiments. However, these exemplary embodiments are for illustrative purposes only, and the scope of the present invention is not intended to be limited thereto.

### Example 1: Fabrication of organ-on-a-chip with inverted micro-weir structure

An organ-on-a-chip of the present invention was fabricated using a polydimethylsiloxane (PDMS) elastomer and a slide glass.

A mold that had completed PR patterning was prepared. In addition, a PDMS silicone base elastomer and a curing agent (SYLGARD 184 silicone elastomer kit, Dow Corning) were mixed at a ratio of 10:1, and then bubbles generated during the mixing were eliminated using a vacuum system of a plasma processing system (CUTE, FEMTO Science). Thereafter, the mixture was poured onto the SU-8 mold and solidified by heating on a hot plate at 80°C for 10 hours. The solidified product was released from the mold, followed by cutting according to the appropriate size of a chip, and then punched out to form cell and hydrogel mixture inlet and outlet, and media storage and inlet and outlet according to the size of each by using cylindrical biopsy punches with diameters of 1.2 mm and 8 mm, thereby fabricating a microfluidic device.

The fabricated microfluidic device, which was a PDMS-based microfluidic device, was immersed in a sonicator containing isopropyl alcohol and washed by sonication, and then completely dried by a nitrogen air-gun.

The PDMS-based microfluidic device that had been washed and a slide glass were dried in a dry oven at 80°C for 10 minutes and then sterilized by exposure to ultraviolet light for 30 minutes. Finally, the PDMS-based microfluidic device and the slide glass were placed in a plasma processing system and bonded to each other through oxygen plasma treatment, thereby fabricating an organ-on-a-chip. The fabricated organ-on-a-chip was placed on a 35-mm petri dish and, for enhancing the adhesion resulting from the plasma treatment, was heated on a hot plate, which had been heated to 80°C, for 30 minutes.

The overall size of the organ-on-a-chip fabricated as described above was 2.7 cm in width and 2 cm in length, and the height of all the channel microstructures in the chip was 50 µm. The height except for inverted micro-weir structures was 5 µm. The width of cell channels was 600 µm, which was approximately three times the diameter of in vivo myocardial bundles, about 200 µm.

The width of media channels was 30 µm, which was approximately three times the diameter of in vivo capillaries, about 10 µm. The media channels were designed to mimic the capillary network situated between myocardial fibers in vivo by being placed at both outermost sides of a cell channel.

An inverted micro-weir structure, which was located on the residue outlet side in the cell channel, was designed such that the inverted micro-weir structure was positioned with a thickness of 60 µm in an arc shape toward the residue outlet, just before the width of the cell channel narrowed, thereby inducing the injected cell-hydrogel mixture to accumulate rather than leave toward the residue outlet, consequently achieving smooth injection and enabling the release of only the pressure occurring during the injection toward the residue outlet. Additionally, inverted micro-weir structures provided in adjacent areas between the cell channel and the media channels were designed such that the inverted micro-weir structures were in a rectangular shape with a thickness of 100 µm and, similarly, the cell-hydrogel mixture was allowed to accumulate rather than leave the cell channel. Each of the inlet and outlet connected to the cell channel had a diameter of 1.5 mm, and the diameter of the media channels connected to media supply chambers and media recovery chambers was 8 mm.

### Example 2: Evaluation of functionality of organ-on-a-chip

### Example 2-1: Organ-on-a-chip containing breast cancer cells

### Example 2-1-1: Fabrication of organ-on-a-chip containing breast cancer cells

As for one set of a media supply chamber and a media recovery chamber included in the organ-on-a-chip of the present invention fabricated in Example 1, 200 µl of media (RPMI 1640 (Gibco) supplemented with 10% fetal bovine serum (FBS, Gibco)) was loaded in each of the chambers. The flow of media was connected between each chamber by using a pipette, and then the media was filled inside the media channel located at both sides to finally form the flow of media. Particularly, it was confirmed that a boundary of the media was formed between the media channel and the cell chamber due to surface tension, with the inverted micro-weir structure serving as a boundary.

Then, 3×10⁴ breast cancer cells (Michigan Cancer Foundation-7, MCF-7) were injected into the cell inlet by using a pipette, and the injected breast cancer cells were injected into the cell chamber by pipetting. Thereafter, it was confirmed that the breast cancer cells were at the boundary of the inverted micro-weir structure, with no cell loss, indicating that the breast cancer cells and the media were successfully injected separately. Subsequently, the cells were cultured in a humidified incubator for 2 days under the conditions of 37°C and 5% CO₂.

### Example 2-1-2: Evaluation of functionality of organ-on-a-chip containing breast cancer cells

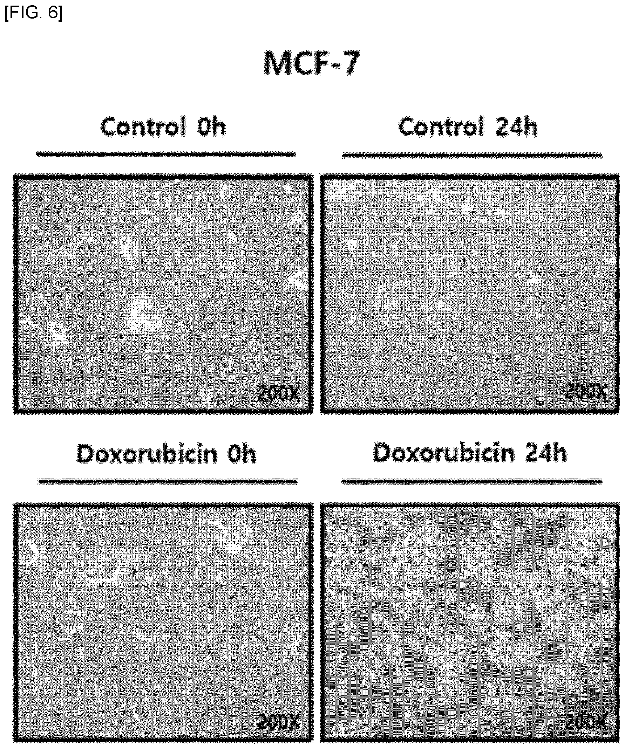

After 100 µM doxorubicin (TOCRIS) was added to the media supply chamber of the organ-on-a-chip containing breast cancer cells fabricated in Example 2-1-1, a change in breast cancer cells in the organ-on-a-chip due to doxorubicin was observed using an optical microscope, and the degree of damage to the breast cancer cells was compared and analyzed with a control group (FIG. 6). An organ-on-a-chip containing breast cancer cells treated without doxorubicin was used as the control group.

FIG. 6 presents images comparing the degree of damage to breast cancer cells (MCF-7) after the breast cancer cells were injected into the organ-on-a-chip provided by the present invention, cultured for 2 days, and then treated with the anticancer drug doxorubicin, compared to the control group.

As seen in FIG. 6, most of the breast cancer cells cultured in the organ-on-a-chip were dead 24 hours after treatment with doxorubicin. However, the cells of the control group were normally cultured without cell death even after 24 hours.

Therefore, the organ-on-a-chip containing breast cancer cells can be used to effectively validate the anticancer activity of doxorubicin against breast cancer cells.

### Example 2-2: Organ-on-a-chip containing cervical cancer cells

### Example 2-2-1: Fabrication of organ-on-a-chip containing cervical cancer cells

As for one set of a media supply chamber and a media recovery chamber included in the organ-on-a-chip of the present invention fabricated in Example 1, 200 µl of media (Dulbecco Modified Eagle Medium (DMEM, Gibco) supplemented with 10% FBS) was loaded in each of the chambers. The flow of media was connected between each chamber by using a pipette, and then the media was filled inside the media channel located at both sides to finally form the flow of media. Particularly, it was confirmed that a boundary of the media was formed between the media channel and the cell chamber due to surface tension, with the inverted micro-weir structure serving as a boundary.

Then, 3×10⁴ cervical cancer cells (HeLa) were injected into the cell inlet by using a pipette, and the injected cervical cancer cells (HeLa) were injected into the cell chamber by pipetting. Thereafter, it was confirmed that the cervical cancer cells (HeLa) were at the boundary of the inverted micro-weir structure, with no cell loss, indicating that the cervical cancer cells (HeLa) and the media were successfully injected separately. Subsequently, the cells were cultured in a humidified incubator for 2 days under the conditions of 37°C and 5% CO₂.

### Example 2-2-2: Evaluation of functionality of organ-on-a-chip containing cervical cancer cells

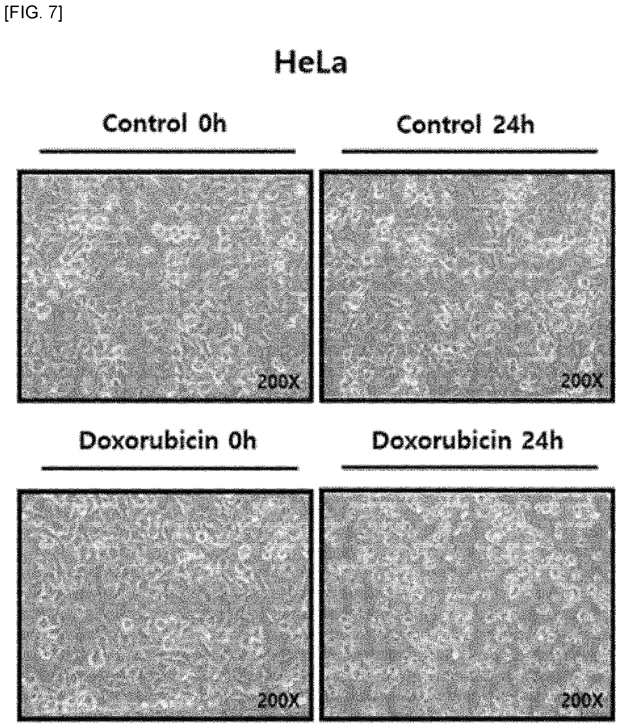

After 100 µM doxorubicin was added to the media supply chamber of the organ-on-a-chip containing cervical cancer cells (HeLa) fabricated in Example 2-2-1, a change in cervical cancer cells in the organ-on-a-chip due to doxorubicin was observed using an optical microscope, and the degree of damage to the cervical cancer cells was compared and analyzed with a control group (FIG. 7). An organ-on-a-chip containing cervical cancer cells treated without doxorubicin was used as the control group.

FIG. 7 presents images comparing the degree of damage to cervical cancer cells (HeLa) after the cervical cancer cells were injected into the organ-on-a-chip provided by the present invention, cultured for 2 days, and then treated with the anticancer drug doxorubicin, compared to the control group.

As seen in FIG. 7, most of the cervical cancer cells cultured in the organ-on-a-chip were dead 24 hours after treatment with doxorubicin. However, the cells of the control group were normally cultured without cell death even after 24 hours.

Therefore, the organ-on-a-chip containing cervical cancer cells can be used to effectively validate the anticancer activity of doxorubicin against cervical cancer cells.

### Example 2-3: Organ-on-a-chip containing liver cancer cells

### Example 2-3-1: Fabrication of organ-on-a-chip containing liver cancer cells

As for one set of a media supply chamber and a media recovery chamber included in the organ-on-a-chip of the present invention fabricated in Example 1, 200 µl of media (Minimum Essential Media Eagle (MEM, high-glucose, Gibco) supplemented with 25 mM HEPES (4-(2-hydroxyethyl)-1-piperazineethanesulfonic acid, Gibco), 25 mM NaHCOs (Sodium bicarbonate, Sigma-Aldrich), 10% (v/v) FBS, and P/S (100Uml-1 penicillin/100Uml-1 streptomycin, Sigma-Aldrich Co., MO)) was loaded in each of the chambers. The flow of media was connected between each chamber by using a pipette, and then the media was filled inside the media channel located at both sides to finally form the flow of media. Particularly, it was confirmed that a boundary of the media was formed between the media channel and the cell chamber due to surface tension, with the inverted micro-weir structure serving as a boundary.

Then, 3×10⁴ liver cancer cells (HepG2) were injected into the cell inlet by using a pipette, and the injected liver cancer cells (HepG2) were injected into the cell chamber by pipetting. Thereafter, it was confirmed that the liver cancer cells (HepG2) were at the boundary of the inverted micro-weir structure, with no cell loss, indicating that the liver cancer cells (HepG2) and the media were successfully injected separately. Subsequently, the cells were cultured in a humidified incubator for 2 days under the conditions of 37°C and 5% CO₂.

### Example 2-3-2: Evaluation of functionality of organ-on-a-chip containing liver cancer cells

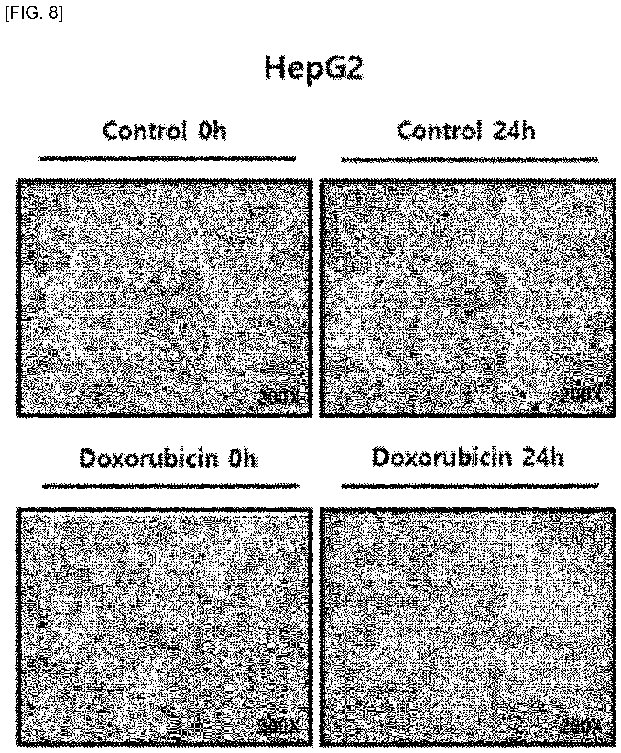

After 100 µM doxorubicin was added to the media supply chamber of the organ-on-a-chip containing liver cancer cells (HepG2) fabricated in Example 2-3-1, a change of liver cancer cells in the organ-on-a-chip due to doxorubicin was observed using an optical microscope, and the degree of damage to the liver cancer cells was compared and analyzed with a control group (FIG. 8). An organ-on-a-chip containing liver cancer cells treated without doxorubicin was used as the control group.

FIG. 8 presents images comparing the degree of damage to liver cancer cells (HepG2) after the liver cancer cells were injected into the organ-on-a-chip provided by the present invention, cultured for 2 days, and then treated with the anticancer drug doxorubicin, compared to the control group.

As seen in FIG. 8, most of the liver cancer cells cultured in the organ-on-a-chip were dead 24 hours after treatment with doxorubicin. However, the cells of the control group were normally cultured without cell death even after 24 hours.

Therefore, the organ-on-a-chip containing liver cancer cells can be used to effectively validate the anticancer activity of doxorubicin against liver cancer cells.

To sum up the results of Examples 2-1 to 2-3, the organ-on-a-chip provided by the present invention can effectively validate the activity of an anticancer drug by culturing various solid cancers, such as breast cancer, cervical cancer, and liver cancer under conditions similar to in vivo conditions.

While the present invention has been described with reference to the particular illustrative embodiments, a person skilled in the art to which the present invention pertains can understand that the present invention may be embodied in other specific forms without departing from the technical spirit or essential characteristics thereof. In this regard, the embodiments described above should be understood to be illustrative rather than restrictive in every respect. The scope of the invention should be construed that the meaning and scope of the appended claims rather than the detailed description and all changes or variations derived from the equivalent concepts fall within the scope of the present invention.

### Explanation of reference numerals

1: PDMS-based microfluidic device
2: slide glass
3: media supply chamber
4: media recovery chamber
5: cardiomyocyte inlet
6: residue outlet
7: media channel
8: cell chamber
9: inverted micro-weir structure located in adjacent area between cell channel and media channel
10: inverted micro-weir structure located in adjacent area between cell chamber and residue outlet

## Claims

1. An organ-on-a-chip, comprising:
(a) a media supply section comprising: a media supply chamber; a media recovery chamber; and a media channel communicating with each of the chambers;
(b) a cell supply section comprising: a cell inlet provided at one end; a residue outlet provided at the other end; and a cell chamber provided between the cell inlet and the residue outlet and extending adjacent to the media channel in the same direction; and
(c) an inverted micro-weir structure provided in an area selected from the group consisting of an adjacent area between the media channel and the cell chamber, an adjacent area between the cell chamber and the residue outlet, and a combination thereof.

2. The organ-on-a-chip of claim 1, wherein the upper end of the inverted micro-weir structure is combined with the upper end of the adjacent area, and the lower end of the inverted micro-weir structure is spaced from the bottom of the adjacent area, thereby allowing the passage of a liquid component through the spaced site while preventing the passage of cells or cured cells therethrough.

3. The organ-on-a-chip of claim 2, wherein the spaced site has a gap of 5 to 20 µm.

4. The organ-on-a-chip of claim 1, wherein the cross-section of the inverted micro-weir structure is rectangular or arc-shaped.

5. The organ-on-a-chip of claim 1 or 2, wherein the inverted micro-weir structure, when provided in the adjacent area between the media channel and the cell chamber, extends along the adjacent area in the same direction and allows the inter-flow of culture media between the media channel and the cell chamber through the spaced site under the lower end.

6. The organ-on-a-chip of claim 1 or 2, wherein the inverted micro-weir structure, when provided in the adjacent area between the cell chamber and the residue outlet, is in the form of an arc curved toward the residue outlet, and allows the discharge of residue from the cell chamber to the residue outlet through the spaced site under the lower end.

7. The organ-on-a-chip of claim 1, wherein the media supply chamber stores media for cell culture, which is to be supplied to the cell chamber through the media channel and the inverted micro-weir structure.

8. The organ-on-a-chip of claim 1, wherein liver cells are further cultured in the media supply chamber for evaluation of drug efficacy and toxicity.

9. The organ-on-a-chip of claim 1, wherein the media channel is a media flow path from the media supply chamber to the media recovery chamber.

10. The organ-on-a-chip of claim 1, wherein the media channel has a width of 20 to 40 µm.

11. The organ-on-a-chip of claim 1, wherein the cell inlet serves to introduce a cell mixture to be supplied to the cell chamber therethrough.

12. The organ-on-a-chip of claim 11, wherein the cell mixture contains cells and a hydrogel for assisting the flow and fixation of the cells.

13. The organ-on-a-chip of claim 1, wherein the cell chamber serves as a culture container in which cells contained in a cell mixture supplied through the cell inlet are arranged and fixed and the fixed cells are cultured.

14. The organ-on-a-chip of claim 13, wherein the fixation of the cells is performed by curing a hydrogel contained in the cell mixture.

15. The organ-on-a-chip of claim 13, wherein the culturing of the cells is performed such that the media supplied from the media channel through the spaced site formed between the lower end of the inverted micro-weir structure and the bottom of the adjacent area is supplied to the cells fixed in the cell chamber.

16. The organ-on-a-chip of claim 13, wherein the cells to be cultured are selected from the group consisting of cancer cells, cardiomyocytes, smooth muscle cells, striated muscle cells, kidney cells, liver cells, epithelial cells, nerve cells, stem cells, vascular endothelial cells, bone cells, scalp cells, spleen cells, lung cells, oral cells, skin cells, hair follicle cells, brain cells, spinal cord cells, and combinations thereof.

17. The organ-on-a-chip of claim 16, wherein the cancer cells to be cultured are breast cancer cells, cervical cancer cells, or liver cancer cells.

18. The organ-on-a-chip of claim 1, wherein the cell chamber has a width of 20 to 2000 µm.

19. The organ-on-a-chip of claim 1, wherein the residue outlet serves to discharge contaminants, generated from the cells that are fixed and cultured in the cell, to the outside.

20. The organ-on-a-chip of claim 1, wherein the organ-on-a-chip comprises one media supply section and one cell supply section.

21. The organ-on-a-chip of claim 1, wherein the organ-on-a-chip comprises multiple media supply sections and one cell supply section.

22. The organ-on-a-chip of claim 21, wherein the media supply sections are arranged at both sides of the cell supply section.

23. The organ-on-a-chip of claim 1, wherein the organ-on-a-chip comprises one media supply section and multiple cell supply sections.

24. The organ-on-a-chip of claim 1, wherein the organ-on-a-chip comprises multiple media supply sections and multiple cell supply sections.

25. The organ-on-a-chip of claim 1, wherein the organ-on-a-chip comprises: two media supply chambers 3; two media recovery chambers 4; two media channels 7; one cell inlet 5; one residue outlet 6; one cell chamber 8 adjacent to the media channels; and inverted micro-weir structures 9 and 10 provided in adjacent areas between the cell chamber and the media channels and in an adjacent area between the cell chamber and the residue outlet.

26. The organ-on-a-chip of claim 25, wherein the two media channels 7 are arranged at both sides of the cell chamber 8.

27. A method for evaluating the efficacy of a drug on cells, the method comprising:
(a) culturing cells contained inside a cell chamber included in the organ-on-a-chip of any one of claims 1 to 26;
(b) administering a desired drug to a media supply chamber included in the organ-on-a-chip; and
(c) measuring a change in the cultured cells.

28. The method of claim 27, wherein the change in the cells is selected from the group consisting of whether cultured cells are damaged, whether cultured cells are grown, whether a secreted component is changed, whether the expression level of a gene is changed, whether the level of a protein is changed, and a combination thereof.

29. The method of claim 27, wherein the cells are cancer cells, and the change in the cancer cells is whether cultured cells are damaged.

30. The method of claim 29, wherein the cancer cells are breast cancer cells, cervical cancer cells, or liver cancer cells.
